# EUROPEAN PATENT SPECIFICATION

(11) **EP 1 372 673 B1**
(45) Date of publication and mention of the grant of the patent: **23.04.2008**
(21) Application number: 02715032.5
(22) Date of filing: 01.03.2002
(51) Int. Cl.: A61K 31/74, A61P 1/00, A61P 3/00, A61P 3/04, A61P 3/06, A61P 3/10

(54) **THE USE OF NON-DIGESTIBLE POLYMERIC FOAMS TO SEQUESTER INGESTED MATERIALS, THEREBY INHIBITING THEIR ABSORPTION BY THE BODY**
VERWENDUNG UNVERDAULICHER, POLYMERER SCHÄUME ZUM ABKAPSELN EINGENOMMENER STOFFE, UND DADURCH VERHINDERUNG IHRE AUFNAHME DURCH DEN KÖRPER
UTILISATION DE MOUSSES POLYMERES NON DIGESTIBLES POUR SEQUESTRER DES MATIERES INGEREES DANS LE BUT D'INHIBER LEUR ABSORPTION PAR LE CORPS

(30) Priority: 19.03.2001 US 277058 P
(43) Date of publication of application: 02.01.2004
(73) Proprietor: THE PROCTER & GAMBLE COMPANY, Cincinnati, Ohio 45202 (US)
(72) Inventor: HIRD, Bryn, Cincinnati, OH 45247 (US); JANDACEK, Ronald, James, Cincinnati, OH 45231 (US)
(74) Representative: Clemo, Nicholas Graham
(86) International application number: PCT/US2002/006529
(87) International publication number: WO 2002/074343

(56) References cited:
- EP-A- 0 901 792
- EP-A- 1 214 934
- WO-A-01/17377
- WO-A-98/51408
- DE-A- 19 942 365
- US-A- 5 650 222
- US-A- 6 048 908
- MARKOVA E A: "TREATMENT OF ALIMENTARY OBESITY WITH OXYGEN FOAM" VRACHEBNOE DELO, vol. 6, 1971, pages 106-108, XP001120225 ISSN: 0049-6804

## Description

### FIELD OF THE INVENTION

The present invention relates to compositions comprising an open-celled polymeric foam wherein the compositions are useful for sequestering lipophilic materials present in the gastrointestinal tract, thereby inhibiting the absorption of such lipophilic materials by the body. The invention further relates to compositions comprising the open-celled polymeric foam wherein the compositions are useful for ameliorating side effects associated with the use of lipase inhibitors. This invention further relates to compositions comprising an open-celled polymeric foam wherein the compositions are useful for the purpose of sequestering aqueous and/or hydrophilic materials present in the gastrointestinal tract, thereby ameliorating diarrhea. This invention additionally relates to kits comprising the compositions and to their non-therapeutic use.

### BACKGROUND OF THE INVENTION

Approximately one third of Americans aged 20 to 74 are considered to be obese, and approximately half of Americans in this age group are considered to be overweight. Obesity is also considered to be a growing problem in other industrialized countries and in developing countries where large numbers of people have become accustomed to Western-influenced high-caloric diets. It has been estimated that obesity contributes to 50% of chronic diseases in Western societies and is responsible for approximately 70% of preventable deaths in the U.S.A. Health care costs associated with obesity are substantial. As a result of these factors, the development of compositions to effect weight-loss is the subject of significant commercial interest.

Approaches to weight-control include appetite suppressants, reduced-caloric diets, exercise regimens, surgical procedures and the like. A variety of compositions for weight-control have been developed. Desired characteristics for such products include the lack of undesirable side-effects, high efficacy, convenient dosage regimens, and low cost. Drugs developed to treat obesity may have undesirable side-effects, may be available only under medical supervision, and may be relatively expensive. Other products such as those with high fiber content may require inconveniently large doses to be effective.

One method of inhibiting the digestion and/or metabolism of dietary lipids is *via* administration of a suitable non-absorbable material to bind or sequester the lipids. For example, U.S. Patent 4,223,023, Furda, issued September 16, 1980, describes the ingestion of chitosan to bind fatty acids and prevent their utilization. Similarly, U.S. Patent 5,453,282, Kanauchi et al., issued September 26, 1995, describes dietary lipid absorption-inhibiting agents comprising a mixture of chitosan and ascorbic acid or a salt thereof. However, the efficacy of chitosan in increasing fat excretion is relatively low, requiring impracticably large doses to be effective as a dietary weight-control supplement. (See, for example, Lengsfeld et al., Obesity Research, Vol. 7, Suppl. 1, Nov. 1999). Certain fat-imbibing polymer particles are described in U.S. Patent 4,432,968 Page et al., issued February 21, 1984. Effective doses exemplified are about ≥1% of the diet. Fat-binding polymers are also described in WO 99/34787, Mandeville et al., published July 15, 1999. All of the materials exemplified in this application have nitrogen-containing functional groups which may be active in binding of bile acids and/or fatty acids. Relatively high doses (≥2% of the diet) are utilized to increase the amount of fat excreted in a rat model. Similarly, U.S. Patent 3,980,968, Ingleman et al., issued September 14, 1976, describes certain solid network *(i.e.,* crosslinked) polymers containing amino groups for binding bile acids. Solid crosslinked polyurethane polymers which form a gel in the presence of water and which are capable of binding cholesterol and lipids have been described as in U.S. Patent 4,340,699, Grouiller, issued July 20, 1982.

Another approach to inhibiting the digestion and/or metabolism of dietary lipids is to utilize compounds which inhibit the activity of certain enzymes necessary for digestion of lipids. Polymers which inhibit the action of pancreatic lipase are described in U.S. Patent 3,923,976, Fields and Johnson, issued December 2, 1975 and U.S. Patent 4,211,765, Johnson and Fields, issued July 8, 1980. However, the efficacy of these materials in inhibiting lipid digestion is also low, as measured by fat excretion.

Non-polymeric compounds which inhibit the activity of gastrointestinal lipases have also been described. For example, the use of a lipase inhibitor (orlistat; XENICAL®) for the control or prevention of obesity and hyperlipidemia is described in U.S. Patent 4,598,089, Hadvary et al., issued July 1, 1986. However, anal leakage of undigested oil is an adverse side effect often observed in subjects treated with sufficiently large doses of lipase inhibitors to be effective in the treatment of obesity. Several approaches have been described to ameliorate this side-effect. Combining a lipase inhibitor with substantial amounts of water-insoluble crude fiber to increase the inhibition of fat absorption is described in U.S. Patent 5,447,953, Isler et al., issued September 5, 1995. Combining a lipase inhibitor with certain poorly digestible, poorly fermentable hydrophilic and/or hydrocolloidal food grade thickeners and or emulsifiers to reduce anal leakage is described in WO 00/09122, Hug et al., published February 24, 2000. Similarly, combining a lipase inhibitor with chitosan or a derivative or salt thereof to reduce anal leakage is described in US Patent 6,030,953, Bailly et al., issued February 29, 2000. However, at convenient dosage levels, the efficacy of such materials in eliminating anal leakage is relatively low, as evidenced by significant levels of oily fur greasing in rodents.

Yet another approach to inhibiting the digestion and/or metabolism of dietary lipids is to replace digestible lipids in the diet with non-digestible substitutes. For example, U.S. Patent 3,600,186, Mattson and Volpenhein, issued August 17, 1971, describes non-digestible, non-absorbable sugar polyester as substitutes for dietary lipids. However, modification of stool rheology due to high levels of undigested oil may be observed in individuals consuming relatively high levels of certain classes of these compounds, leading to symptoms similar to those experienced by patients treated with relatively high levels of lipase inhibitors.

U.S. Patent 4,005,195, Jandacek, issued January 25, 1977, describes certain anti-anal leakage agents for ameliorating such side effects by stiffening the non-digestible oil. Other agents which ameliorate the symptoms associated with relatively high doses of certain non-digestible oil substitutes are described in U.S. Patent 5,451,416, Johnston et al., issued September 19, 1995; U.S. Patent 5,534,284, Corrigan and Howie, issued July 9, 1996; and U.S. Patent 6,077,556, Letton and Feeney, issued June 20, 2000. However, the use of these agents is indicated with foodstuffs comprising non-digestible lipid substitutes rather than for sequestering digestible lipids.

It is known that non-absorbable .lipophilic materials, such as the non-digestible, sugar polyesters described in the aforementioned U.S. Patent 3,600,186, can affect the absorption of toxic lipophilic compounds into the body. Examples of these toxic materials include DDT, polychlorinated biphenyls (PCB's), phthalate esters, and dioxins. Non-digestible, fats and oils have been shown to reduce by more than 50% the absorption of ¹⁴C-labeled DDT orally gavaged into rats (Volpenhein et al., J. Toxicol. and Environ. Health, Vol. 6, pp. 679 - 683, 1980). This effect is the result of the affinity of orally ingested toxic lipophilic materials for the non-absorbable fat. These materials partition into this non-absorbable lipid sink, and are carried into the colon where they cannot be absorbed by the body. The materials are subsequently excreted in the feces.

It is also known that unabsorbable fats and oils enhance the rate of excretion of lipophilic toxins that are stored in the body (Mutter et al., Toxicol. Appl. Pharm., Vol. 92, pp. 428 - 435, 1988; Gesau, et al., Lancet, Vol. 354, pp. 1266 - 1267, 1999; Moser, G. A., Chemosphere, Vol. 39, pp. 1513 - 1521, 1999). The manner in which these non-absorbable fats and oils effect this increase in excretion is based on the metabolism of lipophilic toxins. These substances enter the body *via* various routes, including inhalation and ingestion, and ultimately the substances are stored in the body's adipose tissue and organs. Some of the stored lipophilic toxins are released into the blood and are carried through the liver and bile duct into the intestine. A significant portion of these toxins in the intestine are re-absorbed by the body and re-enter the blood and tissues. Undigested fats and oils in the intestine reduce the absorption of the toxins into the body by partially dissolving them and carrying them into the colon and the feces before they are re-absorbed.

Reduced absorption and enhanced excretion of lipophilic toxins depend on the intestinal presence of fat and/or oil that is not absorbed. Fat that is not absorbed can be presented to the intestine by the inhibition of pancreatic lipase. Lipase inhibitors effectively produce *in situ* undigested fat and/or oil that can dissolve lipophilic toxins and hasten their elimination from the body. Examples of lipase inhibitors include tetrahydrolipstatin (orlistat; XENICAL®) described in U.S. Patent 4,598,089, Hadvary et al., issued July 1, 1986; lipase inhibitors including 2-amino-4H-3,1-benzoxazin-4-one and its derivatives described in WO 0040247 published July 13, 2000; 2-oxy-4H-3,1-benzoxazin-4-ones and its derivatives described in WO 0040569, published July 13, 2000; 2-thio-4H-3,1-benzoxazin-4-one and its derivatives described in WO 0153278, published July 26, 2001; teasaponin described in Han et al., Int. J. Obes. Relat. Metab. Disord., Vol. 25, pp. 1459 - 1464, 2001; long-chain alpha-keto amides described in Chiou et al., Lipids, Vol. 36, pp. 535 - 542, 2001; extract of Nomame Herba described in Yamamoto et al., Int. J. Obes. Relat. Metab. Disord., Vol. 24, pp. 758 - 764, 2000; chiral alkylphosphonates described in Cavalier et al., Chem. Phys. Lipids, Vol. 100, pp. 3 - 31, 1999; chiral isomers of beta-lactone described in Tomoda et al., Biochem. Biophys. Res. Commun., Vol. 265, pp. 536 - 540, 1999; and Pluronic L-101 described in Comai et al., Int. J. Obes., Vol. 4, pp. 33 - 42, 1980. In addition, polymeric substances that imbibe, entrap, or sequester a portion of dietary fat in the intestine reduce the absorption of lipophilic toxins from the intestine by dissolution of the toxins in the dietary fat that is associated with the polymer. A combination of polymers with lipase inhibitors acts to maximize the unabsorbed fat and therefore increase the incorporation of toxins in the unabsorbed fat that is carried into the feces.

Compositions which create a feeling of satiety or fullness can also be effective as weight control agents, either by themselves, or in conjunction with other methods for weight control. For example, U.S. Patent 4,432,968, Battista, issued August 30, 1983, describes mixtures of edible cellulose fibers and/or colloidal cellulose microfibrils which grow in volume in the stomach to form a gelatinous mass and provide a temporary reduction in appetite by a mechanical rather than systemic action. U.S. Patent 5,603,950, Ratjen et al., issued February 18, 1997, describes certain digestible cohesive sponges which may be compressed and inserted into a capsule. After being set free in the stomach, the sponge expands considerably and does not pass immediately into the following digestive tract, but remains in the stomach to provide a temporary sensation of fullness.

As described above, the use of effective doses of agents which inhibit certain enzymes necessary for lipid digestion; or the use of non-digestible, non-absorbable fat substitutes can lead to significant undesirable symptoms. Known materials which sequester or bind dietary lipids typically have low efficacy, requiring inconveniently large doses to be effective in the prevention or treatment of obesity, or in ameliorating the side effects associated with certain drugs, laxatives and fat-substitutes.

Accordingly, it would be desirable to develop a composition for weight control that: (1) is suitable for ingestion; (2) has minimal undesirable side effects; (3) has high efficacy; (4) has convenient dosage regimens; (5) is broadly applicable to various lipids, lipid substitutes, and other lipophilic materials including toxins; and (6) is relatively inexpensive.

### SUMMARY OF THE INVENTION

The present invention relates to compositions, kits and uses according to the claims.

The foams utilized herein are optionally highly compressible open-celled polymeric foams which may be compacted to substantially reduce the bulk of the foam. After ingestion of the composition, the foam can re-expand in the gastrointestinal tract to induce satiety, thereby reducing appetite.

Compositions useful in the present invention may include components administered concurrently with other materials, or ingested separately as part of a dosing regimen during a treatment period. For example, the compositions herein may optionally comprise one or more substances such as enzyme inhibitors (*e.g.*, lipase inhibitors) or laxative agents, or may be used in conjunction with one or more enzyme inhibitors or laxative agents dosed simultaneously or separately. The compositions can ameliorate or eliminate side effects associated with lipase inhibitors.

The compositions of the present invention may be dosed at predetermined times during the day. For example, the compositions may be dosed at about the time food is consumed or at a time when the subject is dosed with an agent that prevents the digestion or absorption of dietary lipids. The compositions of the present invention may also be incorporated into therapeutic kits for the administration of the compositions concomitant with additional materials such as one or more enzyme inhibitors or laxative agents.

Uses of the present compositions and kits are also set forth herein. In addition to sequestration of lipophilic (or, optionally, aqueous and/or hydrophilic) materials present in the gastrointestinal tract of an animal, the present compositions are useful for reducing the amount of lipid metabolized by an animal; treating a condition selected from obesity, hyperlipidemia, diarrhea, gastrointestinal distress, and combinations thereof; inhibiting anal leakage and/or fecal urgency; inducing satiety; effecting weight loss or weight control; reducing levels of toxic substances in an animal; treating the effects resultant from the administration of enzyme inhibitors; and combinations thereof. These and other advantages of the present invention will be readily apparent based on the disclosure herein.

### BRIEF DESCRIPTION OF THE DRAWINGS

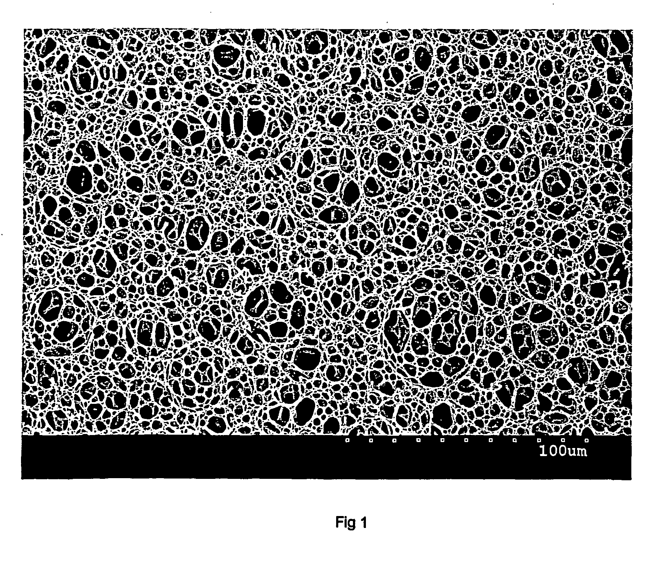

Figure 1 of the drawings is a photomicrograph of a cut section of a polymeric foam useful in the present invention, made from a high internal phase inverse emulsion as Sample 1 of Example 1. A scale is provided in the photomicrograph to enable determination of cell size.

### DETAILED DESCRIPTION OF THE INVENTION

As used herein, the term "safe and effective amount" of a composition is an amount that is effective for sequestering lipids, lipophilic substances, and/or other materials (as appropriate) in an animal, preferably a mammal, and preferably a human, without undue adverse side effects (such as toxicity, irritation, or allergic response), commensurate with a reasonable benefit/risk ratio when used in the manner of this invention. The specific "safe and effective amount" will, obviously, vary with such factors as the particular condition being treated, the physical condition of the treated animal, the size and weight of the treated animal, the duration of treatment, the nature of concurrent therapy (if any), the specific dosage form to be used, other components in the composition, and the dosage regimen desired for the composition.

As used herein, the term "lipid" refers to fats, oils, triglycerides, diglycerides, monoglycerides, other fatty esters (*e.g.,* sucrose fatty acid esters), fatty acids, synthetic oils, mineral oils, grease, petrolatum, and the like.

As used herein, the terms "lipophilic substance", "lipophilic compound" and their plural forms refer to any material which is substantially non-polar in character. Examples of such materials include cholesterol, pesticides such as DDT, tocopherol, terpenes, and the like. Such materials will typically have an octanol/water partition coefficient of greater than 1, as measured according to the method described in Hansch, C. and Leo, A. J., "Substituent Constants for Correlation Analysis in Chemistry and Biology", (1979), John Wiley & Sons, New York.

As used herein, the term "absorb," with reference to a given material, refers to the process of transporting the material, or the breakdown products of the material from the lumen of the intestine into the enterocyte, regardless of whether the material is chemically altered or not, or whether it is metabolized or not. For example, "absorption" of the following materials refers to their transport across the intestinal wall: fats, oils, fatty acids, soaps, monoglycerides, triglycerides, polyglycerides, DDT, PCBs, phthalate esters, dioxins, carbon tetrachloride, cholesterol, and the like. The term "absorbable" refers to a material which is capable of being transported from the lumen through the intestinal wall, either in its chemically unaltered state (*e.g.*, DDT) or after being chemically modified in the gastrointestinal tract (*e.g.*, hydrolysis of fats and oils to form fatty acids and monoacylglycerol). Similarly, the terms "unabsorbable" and "non-absorbable" refer to materials which cannot be transported from the lumen of the intestine into the enterocyte and which cannot be chemically modified in the gastrointestinal tract under normal circumstances to form absorbable materials. Examples of "unabsorbable" or "non-absorbable" materials include, for example, those described in Miller et al., Fundamental Applied Toxicology, Vol. 24, pp. 229 - 237, 1995; and inulin, disclosed in Flamm et al., Critical Rev. Food Science Nutrition, Vol. 41(5), pp. 353 - 362, 2001.

As used herein, the term "non-digestible" means that the referenced material is not susceptible to degradation through the action of digestive enzymes.

As used herein, the term "sequester" used with reference to an open-celled polymeric foam means that a material is held within the pores of the polymeric foam *via* capillary forces, sorption of the material into the polymer itself (*i.e.,* the struts), and/or adsorption onto the surface of the polymer.

### Compositions of the Present Invention

The present invention relates to compositions comprising a non-digestible, non-absorbable, open-celled polymeric HIPE foam and a lipase inhibitor, wherein the compositions are useful for sequestering lipids and/or lipophilic materials present in the gastrointestinal tract (such as, for example, fatty acids, cholesterol, lipid substitutes, toxins, and the like), thereby inhibiting digestion and/or absorption of such materials. The presence of the lipids and/or other lipophilic substances in the gastrointestinal tract may be at least partially due to the action of a lipase inhibitor, and/or to the ingestion of non-digestible lipid-substitutes by an animal. The compositions may therefore be useful for treating certain conditions such as obesity and/or hyperlipidemia, and for effecting weight loss or weight control in an animal. The compositions may also be useful for eliminating or ameliorating the side effects of symptoms which may be associated with the presence of unsequestered lipids and/or certain classes of lipid substitutes in the lower intestine. Examples of such side effects include gastrointestinal distress, fecal urgency, anal leakage, and combinations thereof. The compositions may also be useful for sequestering lipophilic toxins present in the gastrointestinal tract to prevent or reduce their absorption and/or for reducing blood cholesterol levels.

Alternatively or additionally, the present invention relates to compositions comprising a non-digestible, non-absorbable, open-celled polymeric foam wherein the compositions are useful for sequestering aqueous and/or hydrophilic materials present in the gastrointestinal tract, thereby ameliorating symptoms which may be associated with the presence of such materials in the lower intestine. Examples of such side effects include diarrhea and/or loose stools. These symptoms may be due to any of a number of factors, examples of which include the use of laxatives or other agents, illness, and/or food allergies.

Alternatively or additionally, the present invention relates to compositions comprising a non-digestible, non-absorbable, open-celled polymeric foam wherein the compositions are useful for inducing satiety in an animal. The foams utilized herein may be compacted to reduce the bulk of the foam substantially. After ingestion of the composition, the foam can re-expand in the gastrointestinal tract to induce satiety, thereby reducing appetite.

The compositions herein may optionally comprise more substances such as enzyme inhibitors or laxative agents, or may be used in conjunction with more enzyme inhibitors or laxative agents dosed simultaneously or separately. The compositions are particularly useful for ameliorating side effects associated with the use of lipase inhibitors and/or laxative agents.

### Foams of the Present Compositions

The HIPE foams utilized in the present invention are non-digestible and non-absorbable. In addition, the foams are open-celled. As used herein, a foam is "open-celled" if at least about 80% of the cells in the foam structure that are at least 1 µm in size are in unobstructed communication with at least one adjacent cell. Such cells will have intercellular openings or "windows" connecting one cell to the other within the foam structure.

The individual cells in such open-celled foams may be defined by a plurality of mutually connected, three dimensionally branched webs. The individual strands of polymeric material making up these branched webs are referred to herein as "struts." Open-celled foams having a typical strut-type structure are shown by way of example in Figure 1.

Without being bound by theory, the cell size of the foam is believed to be important in determining the ability of the composition to hinder the digestion of sequestered materials. Small-celled foams are believed to sequester materials more effectively than large-celled foams, thereby inhibiting digestion by the gastric fluid.

In order to provide a high level of efficacy, it is desirable that the foams useful in the present invention have a high capacity to sequester or bind materials present in the gastrointestinal tract. For convenient dosage regimens, it is desirable that the effective dose occupies a relatively small volume on ingestion. It is thus desirable that the foams are highly compressible and sufficiently resilient to allow re-expansion of the foam in the gastrointestinal tract after long periods of storage in a highly compressed state. The more compressed the foam upon ingestion, the greater the subsequent volume expansion of that foam is in the gastrointestinal tract, and the greater the efficacy in terms of sequestering capacity for a given volume of ingested material. A high degree of compressibility allows a reduction in bulk and facilitates ingestion to provide convenient dosage regimens.

In order to provide a high capacity and a high degree of compressibility, the foam should have a relatively high void volume. A high void volume is characteristic of low-density foams. Foam density *(i.e.,* in grams of foam per cubic centimeter of foam volume in air) is specified herein on a dry basis in the fully expanded state without any confining pressure. Any suitable gravimetric procedure that will provide a determination of mass of solid foam material per unit volume of foam structure can be used to measure foam density. For example, the ASTM gravimetric procedure described more fully in U.S. Patent 5,387,207, Dyer et al., issued February 7, 1995, is one method that can be employed for density determination.

### HIPE Foams

Preferred polymeric foams useful herein are prepared by polymerization of the oil phase of certain water-in-oil emulsions having a relatively high ratio of water phase to oil phase, commonly known in the art as "HIPE." As used herein, a polymeric foam material which results from the polymerization of such emulsions is referred to herein as a "HIPE foam." HIPE foams comprise a generally hydrophobic, flexible or semi-flexible, nonionic polymeric foam structure of interconnected open-cells.

HIPE foams suitable for use in the present invention and processes suitable for preparing such foams are described in U.S. Patent 5,149,720, DesMarais et al., issued September 22, 1992, U.S. Patent 5,260,345, DesMarais et al., issued November 9, 1993; U.S. Patent 5,268,224 DesMarais et al., issued December 7, 1993; U.S. Patent 5,563,179, Stone et al., issued October 8, 1996; U.S. Patent 5,650,222, DesMarais et al., issued July 22, 1997; U.S. Patent 5,741,518, DesMarais et al., issued April 21, 1998; and U.S. Patent 5,827,909, DesMarais et al., issued October 27, 1998.

### A. Components of the HIPE

HIPE foams may be prepared via polymerization of a HIPE comprising a discontinuous water phase and a continuous oil phase, wherein the ratio of water-to-oil is at least about 10:1, by weight. The water phase generally contains an electrolyte and a water-soluble initiator. The oil phase generally consists of substantially water-insoluble monomers which can be polymerized by free radicals, an emulsifier, and other optional ingredients defined below. The monomers are selected so as to confer the properties desired in the resulting polymeric foam, for example mechanical integrity sufficient for the end use, flexibility, resilience, lipophilic character, and economy. Preferably, the glass transition temperature (Tg) of the resulting foam will be from about -40° to about 90°C so as to confer sufficient flexibility to allow for compression of the foam to reduce its bulk and thereby facilitate ingestion.

### 1. Oil Phase Components of the HIPE

The continuous oil phase of the HIPE comprises monomers that are polymerized to form the solid foam structure and the emulsifier necessary to stabilize the emulsion. In general, the monomers will include from about 20% to about 95%, alternatively from about 45% to about 65%, by weight of at least one substantially water-insoluble monofunctional monomer capable of forming an atactic amorphous polymer having a glass transition temperature (Tg) of about 90°C or lower. This co-monomer is added to lower the overall Tg of the resulting HIPE foam. Exemplary monomers of this type include C₄-C₁₄ alkyl acrylates and C₆-C₁₆ methacrylates such as 2-ethylhexyl acrylate, isobornyl acrylate, n-butyl acrylate, hexyl acrylate, n-octyl acrylate, nonyl acrylate, decyl acrylate, isodecyl acrylate, tetradecyl acrylate, benzyl acrylate, nonyl phenyl acrylate, isobornyl methacrylate, hexyl methacrylate, octyl methacrylate, nonyl methacrylate, decyl methacrylate, isodecyl methacrylate, dodecyl methacrylate, and tetradecyl methacrylate; substituted acrylamides or methacrylamides, such as N-octadecyl (meth)acrylamide; dienes such as isoprene, butadiene, chloroprene, piperylene, 1,3,7-octatriene, beta-myrcene and amyl butadiene; substituted C₄-C₁₂ styrenics such as *p*-n-octyl styrene; vinyl norbornene; and combinations of such monomers.

The oil phase will also comprise from about 5% to about 80%, by weight, of a substantially water-insoluble, polyfunctional crosslinking agent. This co-monomer is added to confer strength to the resulting HIPE foam. Exemplary crosslinking monomers of this type encompass a wide variety of monomers containing two or more activated vinyl groups, such as the divinyl benzenes and analogs thereof. These analogs include *m,p*-divinyl benzene mixtures with ethyl styrene, divinyl naphthalene, trivinyl benzene, divinyl alkyl benzenes, divinyl biphenyls, divinyl phenyl ethers, divinyl ferrocenes, divinyl furans, and the like. Other useful crosslinking agents may be selected from a group derived from the reaction of acrylic acid or methacrylic acid with polyfunctional alcohols and amines. Examples of this group include 1,6-hexanedioldiacrylate, 1,4-butanedioldimethacrylate, trimethylolpropane triacrylate, hexamethylene bisacrylamide, and the like. Other examples of crosslinking monomers include divinyl sulfide, divinyl sulfone, and trivinyl phosphine. Other crosslinkers useful in this regard are well known to those skilled in the art. It should be noted that the weight fraction of the crosslinking component is calculated on the basis of the pure crosslinker in cases wherein the crosslinking monomer is commonly used as a mixture (*e.g.*, divinyl benzene often is a 55% pure mixture with the balance being ethyl styrene). Mixtures of the above crosslinkers may also be employed (*e.g.*, divinyl benzene and 1,6-hexanedioldiacrylate).

Other substantially water-insoluble comonomers may be added to the oil phase in amounts of from 0% to about 70%, alternatively from about 15% to about 40%, by weight, to modify properties in other ways. In certain cases, "toughening" monomers may be desired which impart toughness to the resulting HIPE foam equivalent to that provided by styrene. These include styrenics, such as styrene, 4-*tert*-butyl styrene, and ethyl styrene, and methyl methacrylate. Also included are styrenics and other compounds which may also help reduce the Tg or enhance the strength of the resulting HIPE foam such as *p*-n-octyl styrene. Monomers may be added to form a wettable surface on the HIPE foam struts, or for any other purpose. Other additives, such as fillers, or other materials as may be desired, can also be added to the HIPE prior to curing.

Monomers that contain functional groups may also be employed. For example, monomers with amine groups may be useful in providing foam with enhanced ability to bind fatty acids. Dialkylaminoalkyl (meth)acrylates such as dimethylaminoethyl acrylate are examples of such monomers. Because such functional groups are generally detrimental to emulsion formation and/or stability, monomers may be useful which facilitate the formation of functional groups via chemical modification of the foam after polymerization. For example, an oil phase comprising the *tert-*butyl or cyclohexyl ester of an acrylate, methacrylate, acrylamide, or methacrylamide may be used to make HIPE foam. After curing the foam, the *tert*-butyl or cyclohexyl ester groups may be hydrolyzed under appropriate conditions to yield foam containing the corresponding functional groups. Alternatively, monomers that contain functional groups, or those which facilitate the formation of functional groups may be polymerized or co-polymerized with other monomers prior to incorporation into the oil phase.

### 2. Emulsifier

An emulsifier is necessary for forming and stabilizing the HIPE. The emulsifier is generally included in the oil phase and tends to be relatively hydrophobic in character (see, for example, Williams, J. M., Langmuir, Vol. 7, pp. 1370 - 1377, 1991). Such emulsifiers are advantageously added to the oil phase such that the oil phase comprises from about 1% to about 20% emulsifier, by weight of the oil phase. Emulsifiers that are particularly useful for stabilizing HIPE at high temperatures are preferred. The following discussion sets forth the particularly preferred, oxidatively stable emulsifier compositions.

### 2.1 Primary Emulsifier

The emulsifier component of the oil phase comprises at least a primary emulsifer. Suitable primary emulsifiers are well known to those skilled in the art. Particularly preferred emulsifiers include CRILL-6^{™}, SPAN 20^{™}, SPAN 40^{™}, SPAN 60^{™}, and SPAN 80^{™}. These are nominally esters of sorbitan derived from lauric, myristic, stearic, and oleic acids, respectively. Other preferred emulsifiers include the diglycerol esters derived from monooleate, monomyristate, monopalmitate, and monoisostearate acids. Another preferred emulsifier is diglycerol monooleate (DGMO). Mixtures of these emulsifiers are also particularly useful, as are purified versions of each, specifically sorbitan esters containing minimal levels of isosorbide and polyol impurities.

A preferred emulsifier is described in U.S. Patent 6,207,724, Hird et al., issued March 27, 2001. Such emulsifiers comprise a composition made by reacting a hydrocarbyl substituted succinic acid or anhydride or a reactive equivalent thereof with either a polyol (or blend of polyols), a polyamine (or blend of polyamines) an alkanolamine (or blend of alkanol amines), or a blend of two or more polyols, polyamines and alkanolamines. The lack of substantial carbon-carbon unsaturation renders them substantially oxidatively stable.

### 2.2 Secondary Emulsifier

In addition to these primary emulsifiers, secondary emulsifiers can be optionally included in the emulsifier component. Again, those skilled in the art will recognize that any of a variety of known emulsifiers may be used. These secondary emulsifiers are at least cosoluble with the primary emulsifier in the oil phase. Secondary emulsifiers can be obtained commercially or prepared using methods known in the art. The preferred secondary emulsifiers are ditallow dimethyl ammonium methyl sulfate and ditallow dimethyl ammonium methyl chloride. Wherein these optional secondary emulsifiers are included in the emulsifier component, it is typically at a weight ratio of primary to secondary emulsifier of from about 50:1 to about 1:4, alternatively from about 30:1 to about 2:1.

As is indicated, those skilled in the art will recognize that any suitable emulsifier(s) can be used in the processes for making the foams useful in the present invention. See *e.g.*, U.S. Patent 5,387,207, Dyer et al., issued February 7, 1995 and U.S. Patent 5,563,179, Stone et al., issued October 8, 1996.

The oil phase used to form the HIPE comprises from about 85% to about 98% monomer component and from about 2% to about 15% emulsifier component, all by weight of the oil phase. Preferably, the oil phase will comprise from about 90% to about 97% monomer component and from about 3% to about 10% emulsifier component, all by weight of the oil phase. The oil phase also can contain other optional components. One such optional component is an oil-soluble polymerization initiator of the general type well known to those skilled in the art, such as described in U.S. Patent 5,290,820, Bass et al., issued March 1, 1994.

### 3. Aqueous Phase Components

The discontinuous aqueous internal phase of the HIPE is generally an aqueous solution containing one or more dissolved components. One essential dissolved component of the aqueous phase is a water-soluble electrolyte. The dissolved electrolyte minimizes the tendency of monomers, co-monomers, and crosslinkers that are primarily oil soluble to also dissolve in the aqueous phase.

Any electrolyte capable of imparting ionic strength to the water phase can be used. Preferred electrolytes are mon6-, di-, or trivalent inorganic salts, such as the water-soluble halides (*e.g.*, chlorides), nitrates, and sulfates of alkali metals and alkaline earth metals. Examples include sodium chloride, calcium chloride, sodium sulfate, and magnesium sulfate. For HIPE's that are used to make polymeric foams, calcium chloride is most preferred. Generally, the electrolyte will be utilized in the water phase of the HIPE in a concentration in the range of from about 0.2% to about 40%, alternatively from about 1% to about 20%, and alternatively from about 1 % to about 10%, all by weight of the water phase.

Another component of the aqueous phase is a water-soluble free-radical initiator, as will be known to the art. The initiator can be present at up to about 20 mole percent based on the total moles of polymerizable monomers present in the oil phase. More preferably, the initiator is present in an amount of from about 0.001 to about 10 mole percent based on the total moles of polymerizable monomers in the oil phase. Suitable initiators include ammonium persulfate, sodium persulfate, and potassium persulfate.

### B. Processing Conditions for Obtaining HIPE Foams

HIPE Foam preparation typically involves the steps of: 1) forming a stable high internal phase emulsion (HIPE); 2) curing this stable emulsion under conditions suitable for forming a cellular polymeric structure; 3) compressing and washing the cellular polymeric structure to remove the original residual aqueous phase from the polymeric foam structure and, if necessary, treating the polymeric foam structure with a hydrophilizing surfactant and/or hydratable salt to deposit any needed hydrophilizing surfactant/hydratable salt, and 4) thereafter dewatering this polymeric foam structure.

### 1. Formation of HIPE

The HIPE is formed by combining the aqueous and oil phase components in a ratio ranging from about 8:1 to about 140:1, alternatively from about 10:I to about 75:1, alternatively from about 13:1 to about 65:1, by weight. As discussed above, the oil phase will typically contain the requisite monomers, co-monomers, crosslinkers, emulsifiers, and co-emulsifiers, as well as optional components as may be desired. The aqueous phase will typically contain electrolyte or electrolytes and polymerization initiator or initiators.

The HIPE can be formed from the combined oil and aqueous phases by subjecting these combined phases to shear agitation. Shear agitation is generally applied to the extent and for a time period necessary to form a stable emulsion. Such a process can be conducted in either in batches or in a continuous fashion and is generally carried out under conditions suitable for forming an emulsion where the aqueous phase droplets are dispersed to such an extent that the resulting polymeric foam will have the requisite structural characteristics. Emulsification of the oil and aqueous phase combination will frequently involve the use of a mixing or agitation device such as an impeller.

One preferred method of forming HIPE foam involves a continuous process that combines and emulsifies the requisite oil and aqueous phases. In such a process, a liquid stream comprising the oil phase is formed. Concurrently, a separate liquid stream comprising the aqueous phase is also formed. The two separate streams are provided to a suitable mixing chamber or zone at a suitable emulsification pressure and combined therein such that the desired ratio of aqueous phase to oil phase is achieved.

In the mixing chamber or zone, the combined streams are generally subjected to shear agitation provided, for example, by an impeller of suitable configuration and dimensions, or by any other means of imparting shear or turbulent mixing generally known to those skilled in the art. Shear will typically be applied to the combined oil/water phase stream at an appropriate rate and extent. Once formed, the stable liquid HIPE can then be withdrawn or pumped from the mixing chamber or zone. This preferred method for forming HIPE via a continuous process is described in detail in U.S. Patent 5,149,720, DesMarais et al., issued September 22, 1992. See also, U.S. Patent 5,827,909, DesMarais, issued on October, 27, 1998, which describes an improved continuous process having a recirculation loop for the HIPE. The process also allows for the formation of two or more different kinds of HIPE in the same vessel as disclosed in U.S. Patent 5,817,704, Shiveley et al., issued October 6, 1998. In this example, two or more pairs of oil and water streams may be independently mixed and then blended as required. Alternatively, in-line mixing techniques may be used, such as those described in U.S. Patent Application Serial No. 09/684,037, filed in the names of Catalfamo et al. on October 6, 2000.

### 2. Polymerization/Curing of the HIPE Oil Phase

The HIPE formed will generally be collected in or poured into a suitable reaction vessel, container or region to be polymerized or cured. In one embodiment, the reaction vessel comprises a tub constructed of polyethylene from which the eventually polymerized/cured solid foam material can be easily removed for further processing after polymerization/curing has been carried out to the extent desired. It is usually preferred that the temperature at which the HIPE is poured into the vessel be approximately the same as the polymerization/curing temperature.

The emulsifiers of the present invention are also suitable for stabilizing the HIPE during relatively rapid curing at elevated temperatures. Suitable polymerization/curing conditions will vary, depending upon the monomer and other makeup of the oil and water phases of the emulsion (especially the emulsifier systems used), and the type and amounts of polymerization initiators used. Frequently, however, suitable polymerization/curing conditions will involve maintaining the HIPE at elevated temperatures above about 50°C, alternatively above about 65°C, and alternatively above about 80°C, for a time period ranging from about 20 seconds to about 64 hours, alternatively from about 1 minute to about 48 hours. Conditions which aid in reducing the curing time are discussed in detail in U.S. Patent 5,189,070, Brownscombe et al., issued Feb. 23, 1993 and in U.S. Patent Application Serial No. 09/255,225, filed in the name of DesMarais et al. on February 22, 1999.

A porous water-filled open-celled HIPE foam is typically obtained after curing the HIPE. This cured HIPE foam may be cut or sliced into a sheet-like form. It has been found that such sheets of cured HIPE foam may be readily processed by subsequent treating/washing and dewatering steps useful for modifying foam properties for end use applications. The cured HIPE foam may be cut or sliced to provide a cut thickness in the range of from about 0.08 cm to about 2.5 cm. Alternatively, the foam may be milled, ground, or otherwise comminuted into particles of the desired size and shape.

### 3, Treating/Washing HIPE Foam

The solid polymerized HIPE foam formed will generally be filled with residual water phase material used to prepare the HIPE. This residual water phase material (generally an aqueous solution of electrolyte, residual emulsifier, and polymerization initiator) should be at least partially removed prior to further processing and use of the foam. Removal of this original water phase material will usually be carried out by compressing the foam structure to squeeze out residual liquid and/or by washing the foam structure with water or other aqueous washing solutions. Frequently several compressing and washing steps, for example, from 2 to 4 cycles, will be used.

After the original water phase material has been removed to the extent required, the HIPE foam, if needed, can be treated, for example, by continued washing, with an aqueous solution of a suitable hydrophilizing surfactant and/or hydratable salt.

Optionally, residual surfactant and any other extractable materials can be removed by washing with an appropriate solvent such as 2-propanol, ethanol, or acetone.

### 4. Foam Dewatering

After the HIPE foam has been treated/washed, it will generally be dewatered. Dewatering can be achieved by compressing the foam to squeeze out residual water or other solvent, by subjecting the foam and the liquid therein to temperatures of from about 60°C to about 200°C, or to microwave treatment, by vacuum dewatering or by a combination of compression and thermal drying/microwave/vacuum dewatering techniques. The dewatering step will generally be carried out until the HIPE foam is ready for use and is as dry as practicable. One means of dewatering is described in U.S. Patent Application Serial No. 09/687,280, filed in the names of Weber et al. on October 13, 2000, which describes capillary methods of dewatering HIPE foams. Such capillary dewatering may optionally be followed by a drying step.

### C. HIPE Foam Properties

In addition to being non-absorbable, non-digestible, open-celled foams, preferred HIPE foams useful in the present invention have certain desirable properties. Examples of such properties are detailed below:

### 1. Microstructure

HIPE foam cells will frequently be substantially spherical in shape. The size or diameter of such spherical cells is a commonly used parameter for characterizing foams in general. Since cells in a given sample of polymeric foam will not necessarily be of approximately the same size, an average cell size, *i.e.,* average cell diameter, will often be specified. A method for measuring cell size is disclosed in U.S. Patent 5,563,179, Stone et al., issued October 8, 1996.

The preferred HIPE foams useful in the present invention may have average cell diameters of less than about 150 µm, alternatively from about 5 µm to about 130 µm, alternatively from about 10 µm to about 50 µm, and alternatively from about 15 µm to about 35 µm.

### 2. Density

Preferred HIPE foams useful in the present invention have dry basis density values of less than about 0.1 g/cc, alternatively from about 0.01 g/cc to about 0.1 g/cc, alternatively from about 0.01 g/cc to about 0.05 glee, and alternatively from about 0.01 g/cc to about 0.03 g/cc.

### 3. Glass Transition Temperature (Tg)

An important factor in determining the compressibility of the foam is the flexibility of the polymer from which the foam is comprised. Flexibility is typically characteristic of polymers with relatively low glass transition temperatures. The glass transition temperature (Tg) represents the midpoint of the transition between the glassy and rubbery states of the polymer. Foams comprising one or more polymers with a Tg higher than the temperature of use can be very strong but will tend to be rigid and suffer from permanent damage to the foam structure when compressed to a high degree. Furthermore, foams comprising one or more high Tg polymers typically take a long time to recover to an expanded state after having been stored in a compressed state for prolonged periods. The desired combination of mechanical properties, specifically compressibility and resilience, will necessitate selection between a range of monomer types and levels to achieve the desired end properties.

The Tg of the foams is determined by Dynamic Mechanical Analysis (DMA) using the method described in U.S. Patent 5,817,704, Shiveley et al., issued March 8, 1996. The HIPE foams useful in the present invention will preferably have glass transition temperatures from about - 40°C to about 90°C determined according to this method.

One of ordinary skill in the art will understand that the Tg may be affected by the presence of lipohilic materials which may serve to plasticize the polymer from which the foam is comprised. The measurement of Tg should take into account possible plasticaization under in-use conditions.

### 4. Resilience

The polymer from which the HIPE foam is comprised is preferably sufficiently resilient to allow re-expansion of the foam in the gastrointestinal tract after long periods of storage in a highly compressed state. Typically, this preferred resiliency requires that the polymer be crosslinked to prevent permanent deformation form occurring via stress-relaxation and/or creep. One measure of such permanent deformation is creep recovery. It should be noted that many synthetic polymers are thermoplastic and are thus susceptible to stress relaxation and creep. In such cases, creep recovery can be very slight. For example, a nonwoven polypropylene fiber web of 1 mm thickness loaded to a pressure of 5.1 kPa at 31 °C for 4 hours recovers only slightly after the weight is removed. On the other hand, because they are highly crosslinked, the preferred HIPE foams useful in the present invention provide excellent creep recovery. Suitably, a HIPE foam used in the present invention when similarly loaded to a pressure of 5.1 kPa at 31°C will recover virtually all of its original thickness within a relatively short period, depending on the Tg of the polymer from which the HIPE foam is comprised.

### 5. Specific Surface Area

Another key parameter of the HIPE foams useful in the present invention is their specific surface area, which is determined by both the dimensions of the cellular units in the foam and by the density of the polymer, and is thus a way of quantifying the total amount of solid surface provided by the foam.

Specific surface area is determined by measuring the amount of capillary uptake of a low surface tension liquid (*e.g.,* ethanol) which occurs within a foam sample of known mass and dimensions. A detailed description of such a procedure for determining foam specific surface area *via* the capillary suction method is set forth in the test methods section of in U.S. Patent 5,563,179, Stone et al., issued October 8, 1996. Other similar tests for determining specific surface area can be used with the present foams. Preferred HIPE foams according to the present invention have a specific surface area per unit volume that is greater than about 0.01 m²/cc; alternatively greater than about 0.015 m²/cc, and alternatively greater than about 0.02 m²/cc.

### 6. Surface Hydrophilicity/Lipophilicity

The HIPE foams useful in the present invention will be generally lipophilic to facilitate the sequestering of lipids or other lipophilic materials by the foam in the digestive tract. For example, the internal surfaces of HIPE foam structures may be rendered lipophilic by removal or neutralization of hydrophilizing surfactants and salts left in the foam structure after polymerization. Lipophilic foams are useful for sequestering lipophilic substances present in the digestive tract and/or for stiffening such substances for mitigation of undesirable effects such as anal leakage.

If desired, the foam can be rendered hydrophilic or amphiphilic by treatment with wetting agents. Hydrophilicity may be desired to facilitate sequestering aqueous dietary liquids for mitigation of undesirable effects such as diarrhea.

### Optional Components and Dose Forms of the Present Compositions

The present compositions may be administered concurrently with other materials, or ingested separately as part of a dosing regimen during a treatment period. The present compositions may therefore optionally comprise, for example, one or more drugs, enzyme inhibitors, laxative agents, vitamins, nutrients, excipients, adjuvants, flavorants, diluents, lubricants, sweeteners, antimicrobial agents, and/or the like.

A description of vitamins and nutrients is provided in Handbook of Nonprescription Drugs, 6th Edition, Chapter 10, pp. 141 - 174, 1979. Suitable vitamins and nutrients (including micronutrients) include, but are not limited to, fat soluble vitamins including Vitamins A, D and E; water-soluble vitamins including Vitamins B₁, B₂, B₆, and B₁₂; niacin; beta-carotene; lycopene; bioflavonoids; folic acid; biotin; pantothenic acid; choline; inositol; as well as minerals including iron, calcium, zinc, copper, selenium; trace elements including fluorine, iodine, chromium, cobalt, manganese, molybdenum, nickel, tin, vanadium and silicone; and combinations thereof.

As a further example, the compositions herein may optionally comprise one or more substances such as enzyme inhibitors (*e.g.,* lipase inhibitors) or laxative agents, or may be used in conjunction with one or more enzyme inhibitors or laxative agents dosed simultaneously or separately. To illustrate, one or more of various enzyme inhibitors may optionally be included in the present compositions, or otherwise administered in conjunction with the present compositions (*e.g.,* contemporaneously with the present compositions or at predetermined times relative to administration of the compositions). Lipase inhibitors effectively produce *in situ* undigested fat and/or oil that can dissolve lipophilic toxins and hasten their elimination from the body. Examples of such compounds include tetrahydrolipstatin (orlistat; XENVICAL®) and its derivatives described in U.S. Patent 4,598,089, Hadvary et al., issued July 1, 1986, including those compounds having the following structure: 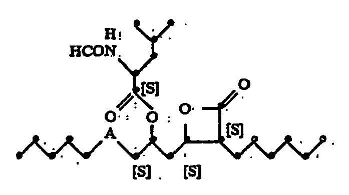 wherein A is the group 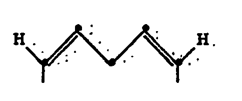 or

-(CH₂)₅-.

Other examples of such lipase inhibitors include 2-amino-4H-3,1-benzoxazin-4-one and its derivatives as described in WO 0040247 published July 13, 2000; 2-oxy-4H-3,1-benzoxazin-4-ones and its derivatives as described in WO0040569, published July 13, 2000; 2-thio-4H-3,1-benzoxazin-4-one and its derivatives as described in WO0153278, published July 26, 2001; teasaponin described in Han et al., Int. J. Obes. Relat. Metab. Disord., Vol. 25, pp. 1459 - 1464, 2001; long-chain alpha-keto amides described in Chiou et al., Lipids, Vol. 36, pp. 535 - 542, 2001; extract of Nomame Herba described in Yamamoto et al., Int. J. Obes. Relat. Metab. Disord., Vol. 24, pp. 758 - 764, 2000; chiral alkylphosphonates described in Cavalier et al., "Chem. Phys. Lipids," Vol. 100, pp. 3 - 31, 1999; chiral isomers of beta-lactone described in Tomoda et al., Biochem. Biophys. Res. Commun., Vol. 265, pp. 536 - 540, 1999; and Pluronic L- 10 described in Comai et al., Int. J. Obes., Vol. 4, pp. 33 - 42, 1980.

A description of suitable excipients and/or other adjuvants is provided in the "Inactive Ingredient Guide" published by the U.S. Food and Drug Administration (see, for example, http://www.fda.gov/cder/drug/iig). Particularly suitable excipients and/or adjuvants comprise sorbitan esters such as sorbitan monolaurate or sorbitan monooleate; cellulose and its derivatives such as carboxymethylcellulose, hydroxypropyl cellulose, cellulose acetate or ethyl cellulose; psyllium and fractions thereof; starch and its derivatives; carbomers; polyethylene glycol and its esters such as PEG stearate; gums such as xanthan gum, karaya gum, gellan gum, or gum arabic; waxes such as paraffin wax or beeswax, carageenan; gelatin; pectin; glycerol (glycerin); polyvinyl acetate phthalate; n-vinyl pyrrolidone; inorganic salts such as calcium salts, magnesium salts, aluminum salts or zinc salts; inorganic oxides such as calcium oxide or magnesium oxide, and combinations thereof.

The composition may be administered in any convenient form including, for example, a capsule, pill, caplet, tablet, chewable tablet, suspension, suppository, or the like. Any method or process for making a suitable dosage form may be employed wherein a mechanical device is employed to compress the foam into solid forms including capsules and tablets that utilize suitable binders and/or coatings that are known to those skilled in the art.

The foams utilized herein are optionally highly compressible open-celled polymeric foams which may be compacted to reduce the bulk of the foam substantially. After ingestion of the composition, the foam can re-expand in the gastrointestinal tract to induce satiety, thereby reducing appetite. Water-soluble or enteric binders or adhesives may be useful for keeping the open-celled polymeric foam in a compressed state to facilitate processing into suitable dosage form such as the capsule, tablet, or pill. After administration of the composition, the foam can re-expand in the gastrointestinal tract upon dissolution of the binder. This expansion may induce satiety in addition to facilitating fat sequestration by the foam.

Any safe and effective amount may be used, but very low doses may not be sufficiently efficacious and high dosages may be inconveniently large to administer. Dosage regimens include those where the diet of the animal comprises from about 0.02 % to about 2%, alternatively from about 0.03% to about 1%, and alternatively from about 0.1% to about 0.5% of the foam, by weight of the diet on a dry basis. As an example, for a human consuming a diet of approximately 600 grams of food per day (on a dry basis), a useful dose would comprise from about 0.12 grams to about 12 grams; alternatively from about 0.18 grams to about 6 grams; and alternatively from about 0.6 to about 3 grams of foam per day. In the alternative, the dosage may be calculated as a percentage of ingested lipid. Useful dosage regimens include those where the foam is administered on a weight basis relative to ingested lipid, for example administering the foam in an amount which is from about 0.15% to about 15%, alternatively from about 0.2% to about 7%, and alternatively from about 0.75% to about 3.75% of the ingested lipid, all on a weight basis. As an example, for a human consuming a diet comprising about 80 grams of lipid per day, a useful dose would comprise from about 0.12 grams to about 12 grams, alternatively from about 0.16 grams to about 5.6 grams, and alternatively from about 0.6 grams to about 3 grams of foam per day.

### Kits of the Present Invention

As has been set forth herein, certain optional components may be included within the compositions of the present invention. In an additional embodiment of the present invention, kits are provided which comprise:
(a) a first composition comprising the non-digestible, non-absorbable, open-celled polymeric HIPE foam described herein; and
(b) a second composition comprising a component selected from the group consisting of vitamins, lipase inhibitors, laxatives, and combinations thereof.

Various vitamins, lipase inhibitors and laxative agents, including those which are preferred for use herein, have been described herein. In accordance with the present embodiment, the first and second compositions will be present in the kits as separate compositions, *e.g.,* as separate dosage forms which are co-packaged, for example, within a containment device.

In yet a further embodiment of the present composition, other kits may comprise:
(a) a composition comprising the non-digestible, non-absorbable, open-celled polymeric HIPE foam described herein; and
(b) information associated with the composition that use of the composition will provide one or more benefits selected from the group consisting of sequestration of lipophlic materials, treatment of gastrointestinal distress, treatment of fecal urgency, treatment of obesity, weight loss, weight control, treatment of hyperlipidemia, treatment of diarrhea, inhibition of anal leakage, reduction of levels of toxic substances, and combinations thereof.
Preferably, such information indicates that one of the benefits described herein will result when the compositions are used in accordance with instructions for use.

In an alternative or additional embodiment, the present kits include aids for improving compliance with regard to administration of compositions of the present invention. In this embodiment, the kits may comprise:
(a) a composition comprising the non-digestible, non-absorbable, open-celled polymeric HIPE foam described herein; and
(b) directions or instructions for use.
For example, such directions or instructions for use may include recommended size and frequency of dose, maximum allowable dose, and/or any contraindications. As a particularly preferred example, such kits may include blister cards wherein each card comprises the total daily dose of the composition to be administered by the user. The blister cards may be divided into sections, usually by perforations wherein each dose section of the blister card comprises a prescribed amount or dose of the composition alone or, for example, with one or more lipase inhibitors either integral to the composition of the present invention or completely separate. See, for example, WO 9822072, published May 28, 1998.

### Non-therapeutic use of the Present Invention

The present non-therapeutic uses are suited for a variety of purposes which are related to the sequestration of various materials including, preferably, lipophilic materials. The uses are therefore suitable for the purpose of sequestering undigested lipids, undigested lipid-substitutes, toxins, and/or other materials present in the gastrointestinal tract. The uses are also suited for treating gastrointestinal distress, treating fecal urgency, treating obesity, treating hyperlipidemia, treating diarrhea, inhibiting anal leakage, reducing levels of toxic substances (in, for example, the gastrointestinal tract), reducing blood cholesterol levels, inducing satiety, effecting weight loss, effecting weight control, and combinations thereof in an animal.

The uses of the present invention comprise administration of the present composition to an animal (preferably a mammal, and most preferably a human). Although the compositions may be administered in a variety of manners which will be well-known to those of ordinary skill, oral administration is preferred. Frequency of administration is not limited, however, the present compositions are typically administered on an infrequent or as-needed basis or may be administered in a more routine manner weekly, daily, or on a more or less frequent basis. For example, the composition may be administered with meals at least once daily, or alternatively at least two to three times daily.

As used herein, the term "administer" with regard to a particular composition means to provide the composition to an animal (including oneself) and/or to direct, instruct, or advise the use of the composition for any purpose (preferably, for a purpose described herein). "Administration" is the corresponding noun. Wherein the administration of one or more of the present compositions is directed, instructed or advised, such direction may be that which instructs and/or informs the user that use of the composition may and/or will provide one or more of the benefits described herein. Non-limiting examples of such instruction or information are set forth herein as part of the description of the present kits.

Administration which is directed may comprise, for example, oral direction (*e.g.*, through oral instruction from, for example, a physician, health professional, sales professional or organization, and/or radio or television media *(i.e.,* advertisement) or written direction (*e.g.*, through written direction from, for example, a physician or other health professional (*e.g.*, scripts), sales professional or organization (*e.g.*, through, for example, marketing brochures, pamphlets, or other instructive paraphernalia), written media (*e.g.*, internet, electronic mail, or other computer-related media), and/or packaging associated with the composition (*e.g.*, a label present on a package containing the composition). As used herein, "written" includes through words, pictures, symbols, and/or other visible descriptors. Such direction need not utilize the actual words used herein, but rather use of words, pictures, symbols, and the like conveying the same or similar meaning are contemplated within the scope of this invention.

### Examples of the Present Invention

### Example 1

HIPE foams which are useful in accordance with the present invention may be prepared by the following processes:

### Sheet Form Process:

General methods for preparing HIPE foams are described in U.S. Patent 5,149,720 DesMarais et al., issued September 22, 1992, U.S. Patent 5,260,345, DesMarais et al., issued November 9, 1993; U.S. Patent 5,268,224, DesMarais et al., issued December 7, 1993; U.S. Patent 5,563,179, Stone et al., issued October 8, 1996; U.S. Patent 5,650,222, DesMarais et al., issued July 22, 1997; U.S. Patent 5,741,518, DesMarais et al., issued April 21, 1998; and U.S. Patent 5,827,909, DesMarais et al., issued October 27, 1998.

A HIPE foam is prepared according to the method described in U.S. Patent 5,650,222, DesMarais et al., issued July 22, 1997, using a water phase comprising 10% calcium chloride and 0.05% potassium persulfate and an oil phase comprising 55 parts EHA, 33 parts DVB-42, 12 parts HDDA, and 6 parts DGMO. The water:oil ratio is 60:1, by weight. As used herein, EHA, DVB-42, HDDA, DGMO, and DTDMAMS are, respectively, as follows:
EHA = 2-ethylhexyl acrylate; available from Aldrich Chemical Co., Milwaukee, WI
DVB-42 = divinyl benzene, 42% purity with 58% ethyl styrene; available from Dow Chemical Corp., Midland, MI
HDDA = 1,6-hexanediol diacrylate; available from Aldrich Chemical Co., Milwaukee, WI
DGMO = Diglycerol Monooleate, available from Danisco Ingredients, Brabrand, Denmark
DTDMAMS = Ditallowdimethyl ammonium methyl sulfate, available from Witco Corp., Greenwich CT.

The HIPE foam is obtained in sheet-form after cutting, washing and dewatering as described in the method in U.S. Patent 5,650,222. This material is designated as Sample 1.

### Small-Scale Process:

Anhydrous calcium chloride (12.0 g) and potassium persulfate (0.150 g) are dissolved in 300 mL of water. This provides the aqueous phase to be used in forming the HIPE.

To a monomer combination comprising 2-ethylhexylacrylate (EHA) (5.50 g), divinylbenzene (of 42% purity with balance being ethyl styrene) (DVB-42) (3.30 g), and 1,6-hexanediol diacrylate (HDDA) (1.20 g) is added a high purity diglycerol monooleate (DGMO) (0.6 g), and ditallowdimethyl ammonium methyl sulfate (DTDMAMS) (0.1g).

A portion of the oil phase (5.00 g) is weighed into a cylindrical high-density polyethylene cup with vertical sides and a flat bottom. The internal diameter of the cup is 70 mm and the height of the cup is 120 mm. The oil phase is stirred using an overhead stirrer equipped with a stainless steel impeller attached to the bottom of a stainless steel shaft 9.5mm (3/8 inch) in diameter. The impeller has 6 arms extending radially from a central hub, each arm with a square cross section 3.5 mm x 3.5 mm, and a length of 27 mm measured from the outside of the shaft to the tip of the arm. The oil phase is stirred with the impeller rotating at 250 to 300 rpm while 300 mL of pre-heated aqueous phase (47°C) is added drop-wise from a jacketed dropping funnel over a period of about 4 minutes. The impeller is raised and lowered within the emulsion during the addition of the aqueous phase so as to achieve a thick high internal phase emulsion (HIPE) with uniform mixing of the components. After all of the aqueous phase has been added, the emulsion is stirred for an additional minute with an impeller speed of about 400 rpm to achieve a thick, uniform HIPE.

The container is covered with a metal lid and placed in a curing oven kept at 65°C for 16 hours. Upon completion of the polymerization/curing, the container is removed from the oven and allowed to cool to room temperature. The cured HIPE foam is removed from the container. The foam at this point is saturated with residual water phase containing dissolved or suspended emulsifiers, electrolyte, and initiator residues. The foam is sliced into disks approximately 1 cm thick using a deli-style meat slicer. Each slice is dewatered by placing it between two pieces of filter paper in a Büchner funnel attached to a filter flask. A vacuum is applied to the filter flask by means of a laboratory aspirator wherein the sample is compressed by placing a rubber dam over the sample and maintaining the system under the vacuum until no more liquid is expressed from the foam. The vacuum is released to provide a disk of dewatered foam.

This material is designated as Sample 2 in the table below. HIPE foam samples with other formulations prepared in a similar fashion are designated as Samples 3 - 5 in the table below. In each case, the amount of oil phase is varied to achieve the desired water-to-oil ratio (W:O ratio):

| Sample | Parts EHA | Parts DVB-42 | Parts HDDA | Parts Styrene | Parts tB-Sty | Parts DGMO | W:O Ratio |
|---|---|---|---|---|---|---|---|
| 2 | 55 | 33 | 12 | 0 | 0 | 8 | 80:1 |
| 3 | 58 | 42 | 0 | 0 | 0 | 8 | 60:1 |
| 4 | 58 | 16 | 0 | 26 | 0 | 6 | 30:1 |
| 5 | 58 | 16 | 0 | 0 | 26 | 6 | 30:1 |

| | | | | | | | |
|---|---|---|---|---|---|---|---|
| wherein: EHA = 2-ethylhexyl acrylate; available from Aldrich Chemical Co. DVB = divinyl benzene, based on 42% purity with 58% ethyl styrene impurity; available from Dow Chemical Corp. HDDA = 1,6-hexanediol diacrylate; available from Aldrich Chemical Co. tB-Sty = 4-tert-Butylstyrene, available from Aldrich Chemical Co., Milwaukee, WI Sty = Styrene, available from Aldrich Chemical Co., Milwaukee, WI | | | | | | | |

### Comminution:

### i) Cut particles

The dewatered foam from the HIPE foam preparation step is washed successively by re-saturating it with water and dewatering it using a Büchner funnel equipped with a rubber dam as described above. The foam is then washed twice with 2-propanol in similar fashion before being dried in a vented vacuum oven for three hours. The dried foam is sliced into cubes approximately 5mm x 5mm x 5mm using a razor blade.

### ii) Ground particulates

The dewatered foam from the foam preparation step is dried in a vented oven at 65°C for three hours, removed from the oven, and allowed to cool to room temperature. Approximately 2 grams of the dried foam are placed in a kitchen blender equipped with a 1.5 L glass container. Examples of suitable blenders are manufactured by Sunbeam Products Inc., Boca Raton, FL (*e.g.*, OSTERIZER®). Water (500 mL) is added to the container and the contents ground for sufficient time to provide a thick slurry comprising foam particles smaller than about 1 mm diameter. Approximately 30 seconds at a low speed is typically sufficient. The slurry is transferred to a Büchner funnel containing appropriate filter paper, and the foam is dewatered using a rubber dam as described above. Several batches of material may be combined and dewatered together. The filter cake is washed by removing it from the Büchner funnel and redispersing the foam particles in distilled water at a ratio of approximately 250 mL water per gram of dry foam. The resultant slurry is filtered and dewatered using a Büchner funnel and rubber dam as described above. The filter cake is washed, filtered and dewatered once more in distilled water, and then twice in isopropanol according to the same procedure. The foam particles are transferred to a large glass tray and spread out into a layer about 1 cm thick, then dried to constant weight in a vented oven at 65°C.

### Example 2

Two groups of rats were matched by weight and placed on a high-fat (17% lard, by weight) diet for 9 days. One of the groups also received the ground particulate HIPE foam from Example 1, Sample 3 at 1.0% of the diet. The diet of the other (Control) group contained 17% lard without any HIPE foam. Total intake and fecal output were measured each day. Pooled feces from the last five days of the feeding period are analyzed for fat content according to AOAC method 954.04, published by AOAC International, Gaithersburg, MD. The results are indicated in the table below.

| % HIPE Foam in Diet | Excreted Fat (as % Ingested Fat) | Std. Error |
|---|---|---|
| 0% (No foam) | 5.73 | 0.28 |
| 1.0% foam | 10.99 | 0.74 |

Normal fat excretion was roughly doubled in the group which was fed HIPE foam. No adverse effects of HIPE foam on the animals were apparent. All rats continued to eat throughout the experiment and maintain normal drinking and grooming. This observation tends to rule out the presence of any illness due to use of the material.

### Example 3

Four groups of rats were matched by weight and placed on a high-fat (17% lard, by weight) diet for 4 weeks. Three of the groups also received ground particulate HIPE foam from Example 1, Sample 3 at 0.25%, 0.5% or 1.0% of the diet. The diet of the fourth (Control) group did not contain HIPE foam. Total intake and fecal output were measured each day during the fourth treatment week. Pooled feces were analyzed for fat content according to AOAC method 954.04, published by AOAC International, Gaithersburg, MD. All three groups receiving HIPE foam showed statistically significant increases in fat excretion relative to the control group during the fourth week of treatment. The results are presented in the table below:

| % HIPE Foam in Diet | Excreted Fat (as % Ingested Fat) | Std. Error |
|---|---|---|
| 0% (Control) | 8.77 | 0.42 |
| 0.25% | 14.21 | 0.71 |
| 0.5% | 17.24 | 0.52 |
| 1.0% | 16.09 | 0.74 |

Normal fat excretion increased by about 50 to about 96% in the groups which received HIPE foam as the dose was increased from 0.25% to 1.0% of the diet. Levels of HIPE foam as low as 0.25% of the diet were quite effective at inhibiting fat absorption.

No adverse effects of HIPE foam on the animals were apparent after four weeks of consumption. All rats continued to eat throughout the experiment and maintain normal drinking and grooming. This observation tends to rule out the presence of any illness due to use of the material.

### Example 4

Three groups of rats were matched by weight and receive a high-fat diet (30% of calories as corn oil) for 9 days. One of the groups also received 400 ppm XENlCAL® as part of the diet The third group received both 400 ppm XENICAL® and 0.5% ground particulate HIPE foam from Example 1, Sample 1 as part of the diet. Total diet intake was measured throughout the study, and fecal output was measured in tail cups fitted to the animals during the last two days of the study. The pooled two-day collection of feces from each animal was analyzed for fat content according to AOAC method 954.04, published by AOAC International, Gaithersburg, MD.

The table below shows the results of fat excretion analyses. Both groups that received XENICAL® excreted significantly more fat than the control group. In addition, the XENICAL® plus HIPE foam group excreted significantly more fat than the group that received only XENICAL ®.

| | | |
|---|---|---|
| Diet Additive | Mean Total 48 hour Lipid Excretion | Excreted Fat (as % Ingested Fat) |
| Control (no XENICAL® or foam) | 0.16 g | 3.9 |
| 400 ppm XET1ICAL® | 2.6 g | 59.1 |
| 400 ppm XENICAL® + 0.5% HIPE foam | 4.6g | 84.8 |

The data indicate an unexpected benefit of combining an open-celled polymeric foam with a lipase inhibitor. The amount of fat excreted as a percent of ingested fat for animals receiving both the foam and the lipase inhibitor together was significantly greater than the combined amount excreted by the animals receiving the foam or the lipase inhibitor separately.

On days 5 and 7 of the study, the appearance of each animal was judged by two observers unaware of the dietary treatment of the animals. These observers assigned numerical values that increased with the amount of oil seen on the fur. A value of 1 was used to describe animals with no oil apparent on their fur. A value of 5 was used to describe animals with more than 90% of their fur coated with oil. Values of 2, 3, or 4, as appropriate, were assigned to animals with intermediate amounts of oil on the fur.

The results of this assessment are shown in the following table:

| Diet Additive | Average Rating |
|---|---|
| Control (no XENICAL® or Foam) | 1.03 |
| 400 PPM XENICAL® | 4.41 |
| 400 ppm XENICAL® + 0.5% HIPE Foam | 1.0 |

The group receiving XENICAL® only was significantly different relative to the other two groups.

### Example 5

Size 00 empty gelatin capsules are obtained from Eli Lilly & Co., Indianapolis, IN. A round-bottomed hole with vertical sides about 8.3 mm in diameter and about 18 mm in depth, is milled into a block of polycarbonate resin using a ball end mill. A gelatin capsule is inserted into the hole and filled with 5mm cubes of HIPE foam from Example 1, Sample 2. The foam is compressed into the bottom of the capsule using a 7.1 mm diameter glass rod with a rounded end. More HIPE foam cubes are added to the capsule and compressed successively until the capsule is filled with compressed foam. The capsule is removed from the polycarbonate resin block and capped to provide a convenient dosage form. Each capsule contains approximately 0.375 grams of HIPE foam.

### Example 6

HIPE foam from Example 1, Sample 1 is compressed into a gelatin capsule together with XENICAL® as described above to provide a convenient dosage form of XENICAL® with the HIPE foam.

### Example 7

HIPE foam from example Example 1, Sample 1 is blended with hydroxypropyl methyl cellulose and compressed in a pill or tablet press to provide a pill or tablet as a convenient dosage form.

## Claims

1. The use of a non-digestible, non-absorbable, open-celled, polymeric HIPE foam in the manufacture of a medicament for sequestering one or more lipophilic materials present in the gastrointestinal tract of an animal.

2. The use of a non-digestible, non-absorbable, open-celled, polymeric HIPE foam in the manufacture of a medicament for treating gastrointestinal distress, treating fecal urgency, treating obesity, treating hyperlipidemia, treating diarrhea, inhibiting anal leakage, reducing levels of lipophilic toxic substances, reducing blood cholesterol levels, and combinations thereof in an animal.

3. The use of Claim 1 or Claim 2 wherein the foam has a density of less than 0.1 g/cc.

4. The use of any preceding claim wherein the medicament further comprises a lipase inhibitor.

5. The use of Claim 4 wherein the lipase inhibitor is selected from 2-amino-4H-3,1-benzoxazin-4-one and its derivatives; 2-oxy-4H-3,1-benzoxazin-4-ones and its derivatives; 2-thio-4H-3,1-benzoxazin-4-one and its derivatives; tetrahydrolipstatin and its derivatives; chiral alkylphosphonates; chiral isomers of beta-lactone; and mixtures thereof

6. The non-therapeutic use of a non-digestible, non-absorbable, open-celled, polymeric HIPE foam for effecting weight control in an animal by sequestering one or more lipophilic materials present in the gastrointestinal tract of the animal.

7. A composition comprising a non-digestible, non-absorbable, open-celled, polymeric HIPE foam and a lipase inhibitor.

8. A kit comprising a non-digestible, non-absorbable, open-celled, polymeric HIPE foam and a lipase inhibitor.

9. The composition of Claim 7 or the kit of Claim 8 wherein the lipase inhibitor is selected from 2-amino-4H-3,1-benzoxazin-4-one and its derivatives; 2-oxy-4H-3,1-benzoxazin-4-ones and its derivatives; 2-thio-4H-3,1-benzoxazin-4-one and its derivatives; tetrahydrolipstatin and its derivatives; chiral alkylphosphonates; chiral isomers of beta-lactone ; and mixtures thereof.

10. The composition of Claim 7 or the kit of Claim 8 wherein the foam has a density of less than 0.1 g/cc.

## Patentansprüche

1. Verwendung eines unverdaulichen, nichtabsorbierbaren, offenzelligen polymeren HIPE-Schaums bei der Herstellung eines Medikaments zur Sequestrierung eines oder mehrerer lipophiler Materialien, die im Magen/Darm-Trakt eines Lebewesens vorhanden sind.

2. Verwendung eines unverdaulichen, nichtabsorbierbaren, offenzelligen polymeren HIPE-Schaums bei der Herstellung eines Medikaments zur Behandlung von Magen/Darm-Beschwerden, Behandlung von Stuhldrang, Behandlung von Fettleibigkeit, Behandlung von Hyperlipidämie, Behandlung von Durchfall, Verhinderung von Analinkontinenz, Senkung der Konzentrationen lipophiler toxischer Substanzen, Senkung von Blutcholesterinwerten und Kombinationen davon bei einem Lebewesen.

3. Verwendung nach Anspruch 1 oder Anspruch 2, wobei der Schaum eine Dichte von weniger als 0,1 g/cm³ hat.

4. Verwendung nach einem der vorstehenden Ansprüche, wobei das Medikament ferner einen Lipaseinhibitor umfasst.

5. Verwendung nach Anspruch 4, worin der Lipaseinhibitor aus 2-Amino-4H-3,1-benzoxazin-4-on und dessen Derivaten; 2-Oxy-4H-3,1-benzoxazin-4-onen und deren Derivaten; 2-Thio-4H-3,1-benzoxazin-4-on und dessen Derivaten; Tetrahydrolipstatin und dessen Derivaten; chiralen Alkylphosphonaten; chiralen Isomeren von beta-Lacton; und Mischungen davon ausgewählt ist.

6. Nichttherapeutische Verwendung eines unverdaulichen, nichtabsorbierbaren, offenzelligen, polymeren HIPE-Schaums zur Ausübung von Gewichtskontrolle bei einem Lebewesen durch Sequestrierung eines oder mehrerer lipophiler Materialien, die im Magen-Darm-Trakt des Lebewesens vorhanden sind.

7. Zusammensetzung, umfassend ein en unverdaulichen, nichtabsorbierbaren, offenzelligen, polymeren HIPE-Schaum und einen Lipaseinhibitor.

8. Kit, umfassend einen unverdaulichen, nichtabsorbierbaren, offenzelligen polymeren HIPE-Schaum und einen Lipaseinhibitor.

9. Zusammensetzung nach Anspruch 7 oder Kit nach Anspruch 8, wobei der Lipaseinhibitor aus 2-Amino-4H-3,1-benzoxazin-4-on und dessen Derivaten; 2-Oxy-4H-3,1-benzoxazin-4-onen und dessen Derivaten; 2-Thio-4H-3,1-benzoxazin-4-on und dessen Derivaten; Tetrahydrolipstatin und dessen Derivaten; chiralen Alkylphosphonaten; chiralen Isomeren von beta-Lacton; und Mischungen davon ausgewählt ist.

10. Zusammensetzung nach Anspruch 7 oder Set nach Anspruch 8, wobei der Schaum eine Dichte von weniger als 0,1 g/cm³ hat.

## Revendications

1. Utilisation d'une mousse polymère à phase interne hautement émulsionnée (HIPE) non digestible, non absorbable, à alvéoles ouvertes, dans la fabrication d'un médicament pour séquestrer un ou plusieurs matériaux lipophiles présents dans l'appareil gastro-intestinal d'un animal.

2. Utilisation d'une mousse polymère à phase interne hautement émulsionnée (HIPE) non digestible, non absorbable, à alvéoles ouvertes dans la fabrication d'un médicament pour le traitement d'un trouble gastro-intestinal, le traitement d'urgence fécale, le traitement de l'obésité, le traitement de l'hyperlipidémie, le traitement de la diarrhée, l'inhibition de fuite anale, la réduction des taux de substances lipophiles toxiques, la réduction des taux de cholestérol dans le sang, et leurs combinaisons chez un animal.

3. Utilisation selon la revendication 1 ou la revendication 2, dans laquelle la mousse a une masse volumique de moins de 0,1 g/cm3.

4. Utilisation selon l'une quelconque des revendications précédentes, dans laquelle le médicament comprend, en outre, un inhibiteur de lipase.

5. Utilisation selon la revendication 4, dans laquelle l'inhibiteur de lipase est choisi parmi 2-amino-4H-3,1-benzoxazin-4-one et ses dérivés ; 2-oxy-4H-3,1-benzoxazin-4-ones et ses dérivés; 2-thio-4H-3,1-benzoxazin-4-one et ses dérivés; tétrahydrolipstatine et ses dérivés ; des alkylphosphonates chiraux ; des isomères chiraux de bêta-lactone ; et leurs mélanges.

6. Utilisation non thérapeutique d'une mousse polymère à phase interne hautement émulsionnée (HIPE) non digestible, non absorbable, à alvéoles ouvertes pour effectuer un contrôle de poids chez un animal en séquestrant un ou plusieurs matériaux lipophiles présents dans l'appareil gastro-intestinal de l'animal.

7. Composition comprenant une mousse polymère à phase interne hautement émulsionnée (HIPE) non digestible, non absorbable, à alvéoles ouvertes et un inhibiteur de lipase.

8. Trousse comprenant une mousse polymère à phase interne hautement émulsionnée (HIPE) non digestible, non absorbable, à alvéoles ouvertes et un inhibiteur de lipase.

9. Composition selon la revendication 7 ou trousse selon la revendication 8, dans laquelle l'inhibiteur de lipase est choisi parmi 2-amino-4H-3,1-benzoxazin-4-one et ses dérivés ; 2-oxy-4H-3,1-benzoxazin-4-ones et ses dérivés ; 2-thio-4H-3,1-benzoxazin-4-one et ses dérivés ; tétrahydrolipstatine et ses dérivés ; des alkylphosphonates chiraux ; des isomères chiraux de bêta-lactone ; et leurs mélanges.

10. Composition selon la revendication 7 ou trousse selon la revendication 8, dans laquelle la mousse a une masse volumique de moins de 0,1 g/cm3.
